# EUROPÄISCHE PATENTSCHRIFT

(11) **EP 1 351 620 B1**
(45) Veröffentlichungstag und Bekanntmachung des Hinweises auf die Patenterteilung: **25.05.2005**
(21) Anmeldenummer: 02704658.0
(22) Anmeldetag: 18.01.2002
(51) Int. Cl.: A61B 19/08

(54) **GEFALTETER MEDIZINISCHER BEZUG ZUR VERWENDUNG IM OPERATIONSSAAL UND VERFAHREN ZUM FALTEN DES BEZUGS**
FOLDED MEDICAL COVER FOR USE IN OPERATION ROOMS AND METHOD FOR FOLDING SAID COVER
GARNITURE MEDICALE PLIEE UTILISEE EN SALLE D'OPERATION, ET PROCEDE DE PLIAGE DE CETTE GARNITURE

(30) Priorität: 18.01.2001 DE 10102001
(43) Veröffentlichungstag der Anmeldung: 15.10.2003
(73) Patentinhaber: Paul Hartmann Aktiengesellschaft, 89522 Heidenheim (DE)
(72) Erfinder: LOHRENGEL, Armin, 89555 Steinheim (DE); KNECHT, Theresia, 73433 Aalen (DE); SPRICK, Ralf, 89522 Heidenheim (DE)
(74) Vertreter: Friz, Oliver
(86) Internationale Anmeldenummer: PCT/EP2002/000476
(87) Internationale Veröffentlichungsnummer: WO 2002/056786

(56) Entgegenhaltungen:
- EP-A- 0 788 777
- DE-C- 19 506 046
- DE-U- 29 807 487
- US-A- 3 747 655
- US-A- 5 379 703

## Beschreibung

Die Erfindung betrifft einen in vorzugsweise ebene Konfiguration gefalteten medizinischen Instrumententischbezug mit den Merkmalen des Oberbegriffs des Anspruchs 1.

Instrumententischbezüge zum Aufziehen oder Überziehen über einen Instrumententisch sind bekannt und beispielsweise in US-A-3,738,405, EP 0 290 738 B1, US-A-3,747,655, DE 195 06 046 C1 und US-A-5,379,703 beschrieben.

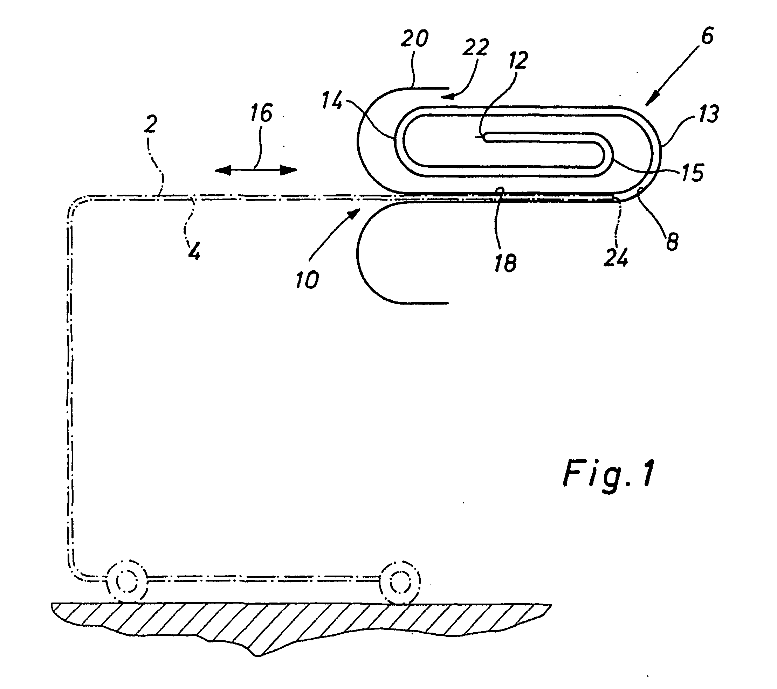

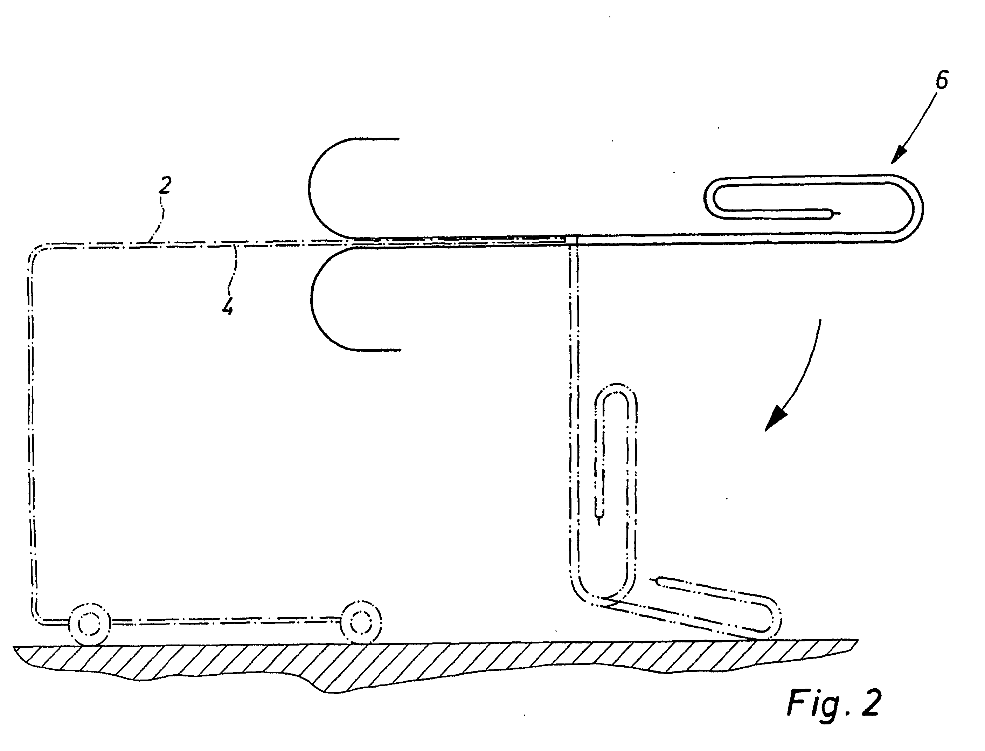

Bei den in EP 0 290 738 B1 und US-A-5,379,703 beschriebenen Instrumententischbezügen ist eine verhältnismäßig lange schlauchförmige Bahn quasi aufgerollt auf eine Seite der Bahn einfach oder mehrfach übereinander gefaltet, so wie dies nachfolgend anhand der den Stand der Technik darstellenden Figuren 1 und 2 beschrieben werden wird. Diese bekannten so gefalteten Instrumententischbezüge erweisen sich in mehrfacher Hinsicht als nachteilig. Beim Aufziehen oder Überziehen oder Überstülpen über einen Instrumententisch wird der Tisch in das offene Ende der schlauchförmigen Bahn des Instrumententischbezugs eingeführt oder anders ausgedrückt, der Instrumententischbezug wird mit seinem offenen Ende über die ebenfalls überwiegend ebene Tischplatte des Instrumententischs übergestülpt. Bei diesem Vorgang gelangt das Tischende dann zu der ersten Umschlagfaltung, und der Benutzer spürt einen Widerstand, der in der Praxis nicht leicht zu überwinden ist. Zumeist ist es erforderlich, dass der Benutzer mit einem Fuß den Instrumententisch gegen Wegrollen sichert oder eine weitere Hilfsperson den Instrumententisch hält. Zumeist ist es erforderlich, um diesen Widerstand der ersten Faltung zu überwinden, dass eine Hilfsperson, die oberhalb des ersten bereits auf den Instrumententisch aufgezogenen Bahnabschnitts liegende einfach oder mehrfach gefaltete oder gerollte Konfiguration anhebt, damit der Benutzer den Bezug weiter auf den Instrumententisch aufschieben kann. Dies bringt aber wiederum die Gefahr von Kontamination des Bezugs mit sich. Ist eine Hilfsperson nicht zur Stelle, so kommt es häufig vor, dass die gefaltete Konfiguration nach unten fällt und mit ihrem zweiten Ende den Boden berührt und hiernach verworfen werden muss.

Mit US-A-3,747,655, welche den Oberbegriff des Anspruchs 1 bildet, wurde das Problem des unkontrollierten Entfaltens oder Aufrollens der gefalteten Konfiguration zwar dadurch gelöst, dass, wie in Figur 2 dieser US-Patentschrift gezeigt, der Bezug zickzack-förmig nach außen hin gefaltet ist. Die jeweiligen Umschlagsfaltungen bilden auf der Außenseite eine Anzahl von stufenförmigen Faltungen, die sich als schmutz- und keimaufnehmende Fugen oder Falten als nachteilig erweisen. Ferner gestaltet sich die Handhabung schwieriger, da ein Benutzer häufig mit seinen Händen nicht in die äußerste eine Handhabungshilfe bildende Umschlagfaltung eingreift, sondern in eine weiter innen liegende. Beim Aufziehen auf einen Instrumententisch streicht der Benutzer dann mit seinen Händen über die Oberfläche des Bezugs oder er muss mehrfach nach- oder umgreifen, um diesen zur Entfaltung zu bringen, was wiederum im Hinblick auf eine Kontamination als nachteilig anzusehen ist. Je größer die Anzahl der nach außen gefalteten Umschläge der schlauchförmigen Bahn ist, umso enger liegen diese gegeneinander an und umso schwieriger ist es, den korrekten Eingriff für die Handhabung des Bezugs beim Aufziehen auf den Instrumententisch zu bekommen.

Hiervon ausgehend liegt der vorliegenden Erfindung die Aufgabe zugrunde, einen Bezug der eingangs beschriebenen Art dahingehend zu verbessern, dass er sich als einfacher handhabbar erweist und auch die vorstehend erwähnten weiteren Nachteile bekannter Bezüge weitestgehend vermieden werden.

Diese Aufgabe wird erfindungsgemäß durch einen Instrumententischbezug mit den Merkmalen des Anspruchs 1 gelöst.

Nach der erfindungsgemäßen Lösung wird also wenigstens ein Zwischenabschnitt in das Innere der schlauchförmigen Bahn eingestülpt oder hineingefaltet, was dazu führt, dass ein die äußere Sichtseite bildender Abschnitt des Bezugs eine sack- oder tütenartige Aufnahme für den restlichen Bezug bildet und keine Faltungen von außen sichtbar sind, die zudem in ungewollter Weise eine höhere Gefahr für eine Kontamination des Bezugs und für Fehlbedienungen bieten. Dadurch, dass der Zwischenabschnitt nach innen eingestülpt oder eingefaltet ist, besteht auch nicht die Gefahr, dass beim Aufziehen des Bezugs auf einen Instrumententisch der Bezug sich ungewollt oder unkontrolliert entfaltet oder entrollt, so wie dies bei eingangs erwähnten bekannten Faltungen passieren konnte, wenn der Widerstand einer Umschlagfaltung ruckartig überwunden werden musste. Es hat sich desweiteren in ganz besonders vorteilhafter Weise gezeigt, dass beim Aufziehen des Bezugs auf einen Instrumententisch, Operationstisch oder eine Extremität, beispielsweise ein Bein eines Patienten, auch kein beachtlicher Widerstand überwunden werden muss, wenn das Tischende gegen das üblicherweise geschlossene Ende des Bezugs stößt, sondern der Bezug entfaltet sich dann quasi widerstandslos und lässt sich so besonders komfortabel über einen Instrumententisch ziehen oder auch auf ein Bein eines Patienten aufziehen oder aufstülpen.

Wenn der nach außen umgefaltete Randabschnitt umlaufend umgefaltet ist, so kann er dadurch den gefalteten Bezug in vorzugsweise eben gefalteter Konfiguration halten. Solchenfalls erstreckt sich der Randabschnitt in Längsrichtung, vorzugsweise über etwa das 0,2 bis 0,5-fache der Länge der gefalteten Konfiguration.

Um eine hinreichende Auszugslänge bei möglichst geringer Länge der gefalteten Konfiguration zu erreichen, erweist es sich als vorteilhaft, wenn mehrere Zwischenabschnitte zwischen dem das zweite Ende bildenden Endabschnitt und dem ersten offenen Ende angeordnet und nach innen in die schlauchförmige Struktur hineingestülpt sind. Ebenfalls erweist es sich im Hinblick auf eine möglichst geringe Länge der gefalteten Konfiguratin als vorteilhaft, wenn der eingestülpte Zwischenabschnitt mit seinem durch die Einstülpung gebildeten entfaltbaren Ende innerhalb der schlauchförmigen Struktur bis nahezu an den Anschlag am zweiten Ende des Bezugs heranreicht. Entsprechendes gilt natürlich auch für den Ausgangspunkt des eingestülpten Zwischenabschnitts, der sich möglichst wenigstens nahezu im Bereich des ersten offenen Endes befinden sollte, um eine optimale Längenausnutzung zu haben.

Der Anschlag am zweiten Ende des Bezugs, welcher verhindern soll, dass der Bezug zu weit auf den Tisch oder die Extremität aufgeschoben wird, könnte in ansich beliebiger Weise, beispielsweise durch einige Laschen oder Streifen am ansonsten offenen zweiten Ende ausgebildet sein, gegen welche das freie Ende des Tischs dann anschlägt und dadurch eine Endposition definiert. Indessen erweist es sich als vorteilhaft, wenn dieser Anschlag dadurch gebildet ist, dass das zweite Ende, beispielsweise durch eine Querschweißung der schlauchförmigen Bahn, ganz geschlossen ausgebildet ist.

Der Bezug weist ferner auf wenigstens einer Seite eine absorbierende Oberfläche, vorzugsweise auf Vliesstoffbasis auf.

Die Erfindung betrifft desweiteren ein Verfahren zum Falten eines Instrumententischbezugs der gattungsgemäßen Art, welches gekennzeichnet ist durch die Verfahrensschritte des unabhängigen Anspruchs 7.

Danach wird zur Herstellung eines jeweiligen Bezugs zunächst ein jeweiliger schlauchförmiger Bahnabschnitt hergestellt, was beispielsweise durch Bilden und Zuführen eines endlosen schlauchförmigen Bahnabschnitts und Abtrennen von einzelnen Längsabschnitten vor oder nach der erfindungsgemäßen Faltung von dieser endlosen Bahn erfolgen kann. Der schlauchförmige Bahnabschnitt kann dabei im Wesentlichen in einer Faltebene orientiert sein und bildet dann eine obere und eine untere Bahn mit jeweiligen Längsenden und jeweiligen Endabschnitten. Erfindungsgemäß wird ein Zwischenabschnitt, der in Längsrichtung zwischen dem das zweite Ende bildenden Endabschnitt und dem ersten offenen Ende angeordnet ist, nach innen in die schlauchförmige Struktur hineingefaltet, so dass der Zwischenabschnitt eine Öffnung zum Einführen des Tischs bildet.

Es wird hierdurch eine erfindungsgemäße gefaltete Konfiguration erreicht, wonach beim Aufziehen auf einen Instrumententisch die Tischplatte in die Öffnung, die von dem in das Innere der schlauchförmigen Struktur hineingefalteten Zwischenabschnitt gebildet und begrenzt ist, eingeführt wird.

Wenn die Konfiguration eben gefaltet ist, so liegen ein oberer und ein unterer Teil des Zwischenabschnitts aneinander an. Es kann aber auch aus irgendwelchen Gründen zwischen diesem oberen und unteren Teil des Zwischenabschnitts eine nicht zum Bezug gehörende Trennlage oder dergleichen zwischengeordnet sein, sollte sich dies als wünschenswert erweisen.

Ausgehend von einem schlauchförmigen Bahnabschnitt, insbesondere mit zwei offenen Enden, wird nach einer Verfahrensvariante nach Anspruch 9 dieser Bahnabschnitt auf einen ersten Rohrkörper aufgezogen, so dass er über den Rohrkörper vorsteht, und mittels eines zweiten Rohrkörpers geringfügig geringeren Durchmessers als der erste wird ausgehend von dem überstehenden Teil des schlauchförmigen Bahnabschnitts der Zwischenabschnitt in die schlauchförmige Struktur hineingestülpt, indem der zweite Rohrkörper unter Mitnahme des Zwischenabschnitts in den ersten hineingeschoben wird.

Nach der Verfahrenvariante nach Anspruch 10 bildet der über den ersten Rohrkörper überstehenden Teil des schlauchförmigen Abschnitts den Zwischenabschnitt, d.h. beim Einstülpen des Zwischenabschnitts gleitet der schlauchförmige Abschnitt nicht gegenüber dem ersten Rohrkörper. Es sind aber auch Verfahrensvarianten denkbar und vorteilhaft, wonach der überstehende Teil des schlauchförmigen Abschnitts von dem zweiten Rohrkörper ergriffen und dann unter Nachziehen des Abschnitts über den ersten Rohrkörper, wobei der Abschnitt über den ersten Rohrkörper gleitet, in diesen hineingestülpt wird.

Es erweist sich weiter als vorteilhaft, wenn der zweite Rohrkörper beim Hineinschieben in den ersten Rohrkörper radial innen geführt wird. Dies kann durch an sich beliebige Führungsmittel, etwa durch einen dritten Rohrkörper erreicht werden. Vorteilhafterweise ist das Führungsmittel von dem ersten Rohrkörper selbst gebildet.

Desweiteren kann ein das Einschieben des zweiten Rohrkörpers begrenzender Endanschlag vorgesehen werden, der vorteilhafterweise von einem nach radial außen gerichteten Bund am zweiten Rohrkörper oder durch ein nach radial innen gerichtetes Anschlagmittel am ersten Rohrkörper gebildet sein kann.

Wie bereits erwähnt, kann der Anschlag, insbesondere nach dem Hineinstülpen des Zwischenabschnitts, vorteilhafterweise durch Schließen des zweiten Endes der schlauchförmigen Struktur, etwa durch Schweißen oder Kleben, hergestellt werden.

Weitere Merkmale, Einzelheiten und Vorteile der Erfindung ergeben sich aus der zeichnerischen Darstellung und nachfolgenden Beschreibung einer Ausführungsform des Standes der Technik und einer erfindungsgemäßen Ausführungsform des Bezugs. In der Zeichnung zeigt:
- Figur 1: eine schematische Darstellung eines zum Stand der Technik gehörenden Instrumententischbezugs beim Aufziehen auf einen Instrumententisch;
- Figur 2: eine weitere schematische Darstellung des in bekannter Weise gefalteten Instrumententischbezugs nach Figur 1 zu einem späteren Zeitpunkt beim Aufziehen auf einen Instrumententisch;
- Figur 3: eine schematische Darstellung einer Ausführungsform des erfindungsgemäß gefalteten Instrumententischbezugs beim Aufziehen auf einen Instrumententisch;
- Figur 4: eine weitere schematische Darstellung des erfindungsgemäß gefalteten Instrumententischbezugs nach Figur 3 zu einem späteren Zeitpunkt beim Aufziehen auf einen Instrumententisch;
- Figur 5: eine schematische Darstellung eines vollständig auf einen Instrumententisch aufgezogenen Bezugs;
- Figuren 6 bis 10: schematische Darstellungen des Faltens eines erfindungsgemäßen Instrumententischbezugs mittels Rohrkörper; und
- Figur 11: eine schematische Darstellung des in ebene Konfigurationen gefalteten Bezugs nach Fig. 6-10.

Figur 1 zeigt in schematischer Andeutung einen Instrumententisch 2 mit einer flächenhaft erstreckten Tischplatte 4 und einen nach dem Stand der Technik gefalteten Instrumententischbezug 6. Der Instrumententischbezug 6 umfasst einen schlauchförmigen Bahnabschnitt 8, der in Figur 1 im Schnitt senkrecht zur Faltebene des Bezugs dargestellt ist. Der schlauchförmige Bahnabschnitt 8 weist ein erstes offenes Ende 10 und ein zweites geschlossenes Ende 12 auf. Zwischen dem offenen Ende 10 und dem geschlossenen Ende 12 ist der schlauchförmige Bahnabschnitt 8 durch drei Faltungen, angedeutet durch Bezugszeichen 13, 14, 15, quer zur Längsrichtung 16 auf sich gefaltet, und zwar derart, dass auf einer ersten Oberseite 18 des schlauchförmigen Bahnabschnitts 8 die gesamte gefaltete Konfiguration zu liegen kommt.

Zum Aufziehen des Instrumententischbezugs 6 greift ein Benutzer mit seinen Händen in eine von einem nach außen umgeschlagenen Randabschnitt 20 gebildete Tasche 22. Er zieht dann den noch in eben gefalteter Konfiguration vorliegenden Bezug 6 mit dessen offenem Ende 10 über das freie Ende 24 der Tischplatte 4 des Instrumententischs 2. Wie in Figur 1 angedeutet, stößt dann das freie Ende 24 gegen die erste Umschlagfaltung 13. Es kommt zu unerwünschtem Widerstand, der sich in der Praxis nur sehr schwer überwinden lässt. Der Instrumententisch 2, der üblicherweise mit Rollen ausgestattet ist, neigt dazu, zurückzuweichen und muss möglicherweise von einer Hilfsperson hieran gehindert werden. Es geschieht dann häufig ein aus Figur 2 ersichtliches und dort skizziertes unkontrolliertes Entrollen oder Entfalten des Instrumententischbezugs 6, und es besteht die Gefahr, dass das verbleibende Ende nach unten fällt und den Boden berührt, so wie dies in Figur 2 angedeutet ist, bevor der Bezug weiter auf die Tischplatte 4 des Instrumententischs aufgezogen werden kann.

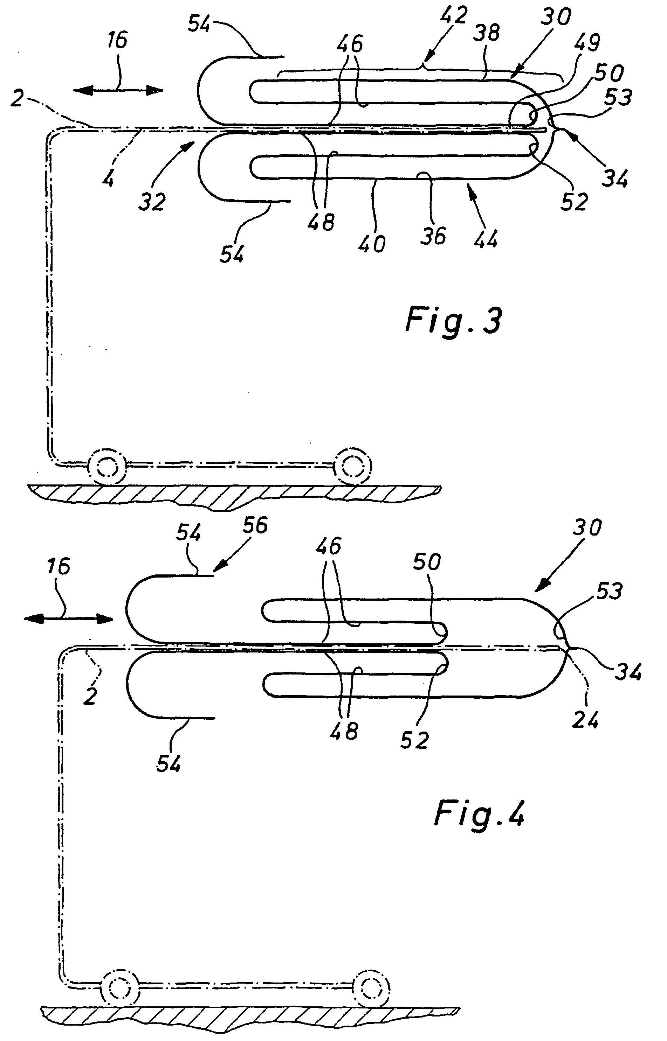

Figur 3 zeigt ebenfalls schematisch eine Ausführungsform eines erfindungsgemäß gefalteten Bezugs 30 für einen Instrumententisch 2, Operationstisch oder auch eine Extremität eines Patienten. Wie bei dem bekannten Bezug weist auch der auf erfinderische Weise gefaltete Bezug 30 ein erstes offenes Ende 32 und ein zweites bei der dargestellten Ausführungsform geschlossenes Ende 34 auf. Der Bezug 30 ist ebenfalls hergestellt aus einem schlauchförmigen Bahnabschnitt 36, der in eben gefalteter Konfiguration einen ersten oberen Bahnabschnitt 38 und einen zweiten unteren Bahnabschnitt 40 bezogen auf die senkrecht zur Zeichnungsebene der Figur 3 verlaufende Faltebene aufweist.

Der erste und zweite Bahnabschnitt 38, 40 weist jeweils einen Endabschnitt 42, 44 auf, welcher das zweite Ende 34 bildet und zudem im dargestellten Ausführungsbeispiel der Figur 3 die äußere Sichtseiten des Bezugs. Der erste und zweite Bahnabschnitt 38, 40 weist auch jeweils einen Zwischenabschnitt 46, 48 zwischen dem jeweiligen Endabschnitt 42, 44 und dem offenen Ende 32 auf. Diese Zwischenabschnitte 46, 48 sind in Längsrichtung 16 nach innen in die schlauchförmige Struktur 36 hineingestülpt oder hineingefaltet, und zwar vorzugsweise so weit, dass ein dabei gebildetes wieder entfaltbares Ende 50, 52 wenigstens nahezu bis an die Innenseite des zweiten geschlossenen Endes 34 des Bezugs 30 heranreicht. Stellt man sich den Bezug nicht wie in Figur 3 eben gefaltet, sondern beispielsweise rohr- oder schlauchförmig vor, so gehen die Zwischenabschnitte 46, 48 ineinander über und bilden eine im Wesentlichen zylindrische Aufnahmeöffnung 49 für den Tisch.

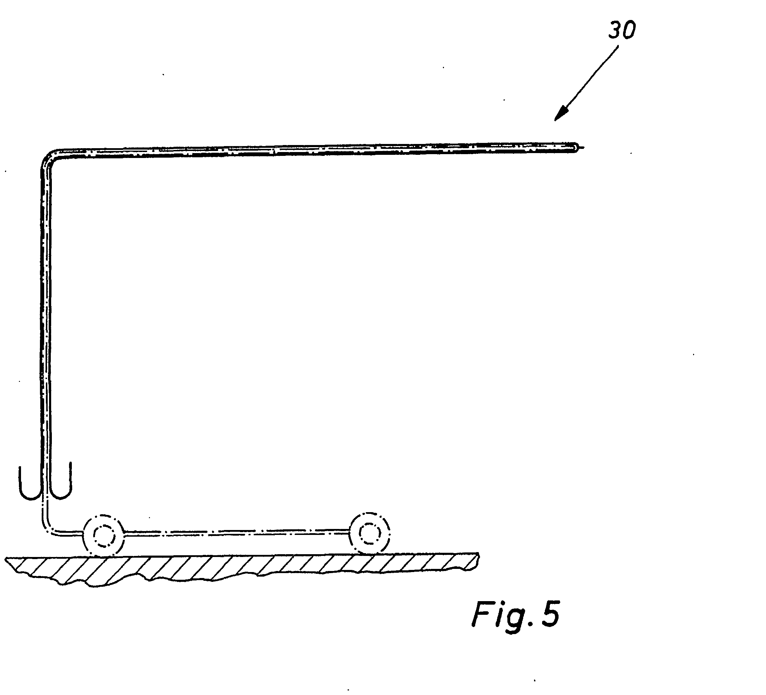

Figur 4 zeigt den Bezug 30 in einem weiter auf den Instrumententisch 2 aufgezogenen Zustand. Die eingestülpten oder eingefalteten Zwischenabschnitte 46, 48 entfalten sich selbstätig und ohne einen bei der Handhabung als unkomfortabel empfundenen Widerstand entgegenzusetzen, wenn das freie Tischende 24 gegen die Innenseite des zweiten geschlossenen Endes 34, welches einen Anschlag 53 bildet, anstößt. Dabei wandern die entfaltbaren Enden 50, 52 in Aufziehrichtung 16 der Figuren 3, 4 nach links, bis die Zwischenabschnitte 46, 48 vollständig entfaltet sind und der Tischbezug dann wieder vollständig entfaltete schlauchförmige Gestalt einnimmt, wie dies aus Figur 5 ersichtlich ist.

Es versteht sich, dass auch mehrere Zwischenabschnitte auf die erfinderische Art und Weise nach innen eingestülpt oder eingefaltet sein können.

Auch bei der erfindungsgemäßen Ausführungsform nach Figuren 3 und 4 ist ein am ersten offenen Ende 32 vorgesehener Randabschnitt 54 umlaufend, also oberhalb und unterhalb der Faltebene, nach außen umgefaltet, um so ein Handhabungshilfsmittel 56 zu bilden. Durch die umlaufende Umfaltung des Randabschnitts 54 wird aber auch der Bezug 30 in der gefalteten Konfiguration gehalten, was sich bei der Handhabung als besonders vorteilhaft erweist, um den Bezug auf das freie Ende eines Instrumententischs oder Operationstischs aufschieben zu können, ohne dass es zu einer ungewollten Entfaltung des Bezugs kommt.

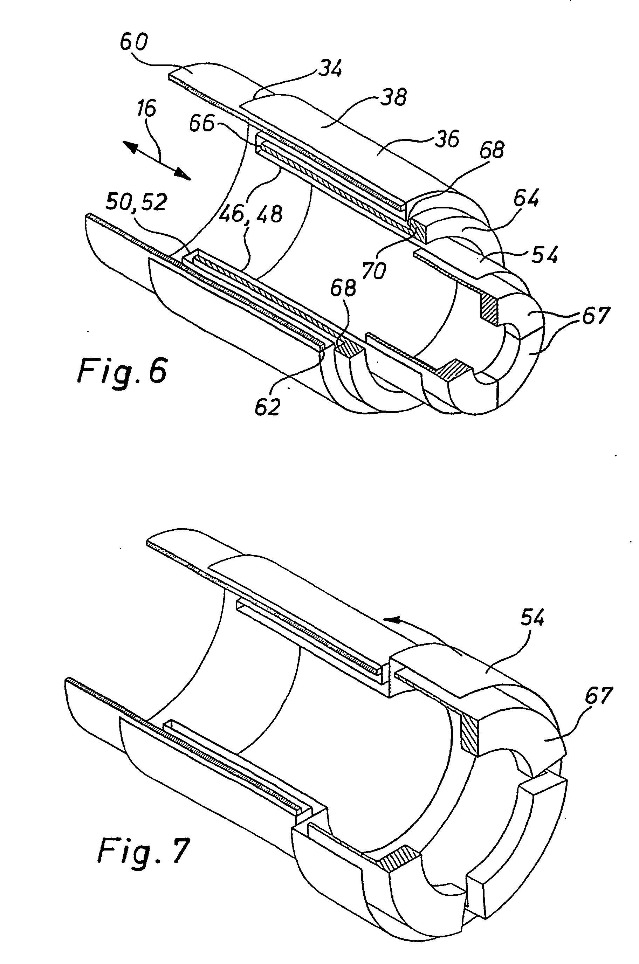

Schließlich zeigen die Figuren 6 bis 10 in schematischer Darstellung die Herstellung der in das Innere des schlauchförmigen Bahnabschnitts 36 gerichteten Hineinstülpung des Zwischenabschnitts 46, 48. Ausgehend von einem in Längsrichtung 16 langgestreckten schlauchförmigen Bahnabschnitt 36 mit zwei offenen Enden 32, 34 wird dieser Bahnabschnitt 36 über eine erste Länge L_{R} auf einen ersten zylindrischen Rohrkörper 60 aufgezogen, so dass er mit seiner restlichen Länge (L - L_{R}) (nicht dargestellt) über ein Ende 62 des Rohrkörpers 60 übersteht. Der überstehende Teil des schlauchförmigen Bahnabschnitts 36 wird sodann durch einen zweiten Rohrkörper 64 hindurch - oder in diesen hineingesteckt, wobei der zweite Rohrkörper 64 einen geringfügig geringeren Außendurchmesser als der Innendurchmesser des ersten Rohrkörpers 60 aufweist, so dass er in den ersten Rohrkörper 60 einsteckbar ist. Der zweite Rohrkörper 64 wird dann unter Mitnahme des überstehenden Teils des Bahnabschnitts 36 in den ersten Rohrkörper 60 hineinbewegt, und dadurch wird der Zwischenabschnitt 46, 48 in die schlauchförmige Struktur hineingestülpt. Ein in den ersten Rohrkörper 60 hineingeschobenes Ende 66 des zweiten Rohrkörpers 64 bildet dabei die in Figuren 3 und 4 dargestellten entfaltbaren Enden 50, 52 des hineingestülpten Zwischenabschnitts 46, 48, welche entsprechend der kreisrunden Öffnungsform des zweiten Rohrkörpers 64 ebenfalls eine Kreisform beschreiben.

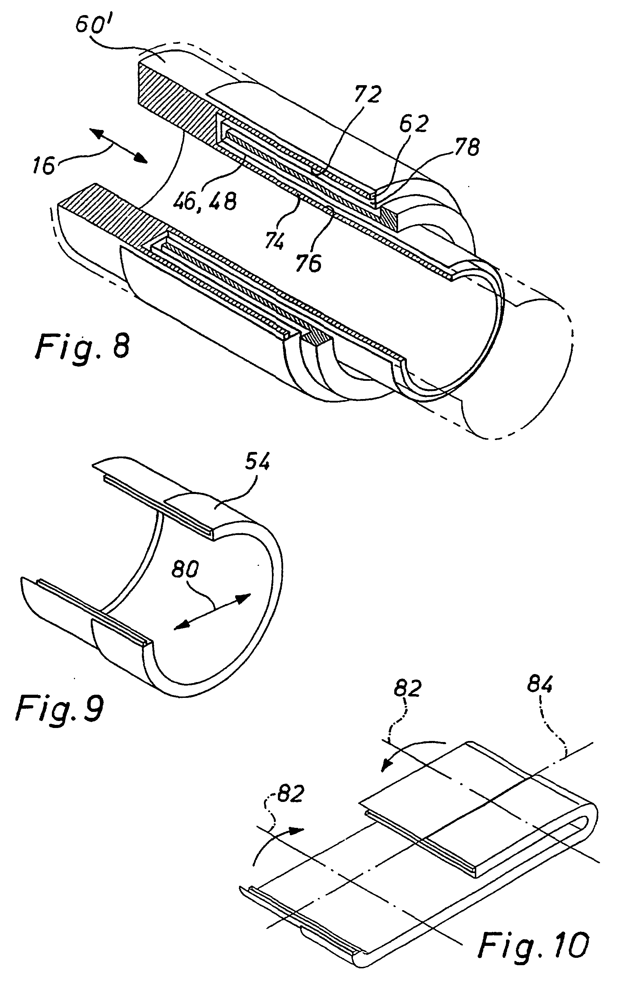

Nachdem der zweite Rohrkörper 64 in entgegengesetzter Längsrichtung 16 wieder aus dem ersten Rohrkörper 60 herausgezogen wurde, kann ein Randabschnitt 54 wie in Figur 7 angedeutet, beispielsweise durch radial bewegliche Segmente 67 (angedeutet in Figuren 6 und 7) nach außen in die in Figur 9 dargestellte Konfiguration umgeschlagen werden. Die Segmente 67 können zum Umschlagen des Randabschnitts 54 in Längsrichtung 16 verfahrbar sein.

Wie aus den Figuren 6 und 7 zu erkennen ist, wird die Einschubbewegung des zweiten Rohrkörpers 64 in den ersten Rohrkörper 60 hinein durch einen Endanschlag 68 begrenzt, der durch einen nach radial außen gerichteten Ringbund 70 am zweiten rohrförmigen Körper 64 gebildet ist. Beim Einschieben des zweiten rohrförmigen Körpers schlägt dieser Ringbund 70 schließlich gegen das Ende 62 des ersten Rohrkörpers 60 an, wodurch eine vorbestimmte Einschublänge gebildet wird.

Figur 8 zeigt die Verwendung eines alternativ gestalteten ersten rohrförmigen Körpers 60'. Dieser weist eine zylindrische konzentrisch zur Längsachse 16 ausgebildete Ausnehmung 72 ausgehend von seinem Ende 62 auf, in welche der zweite Rohrkörper 64 in Längsrichtung 16 unter Mitnahme des Zwischenabschnitts 46, 48 einschiebbar ist. Diese zylindrische Ausnehmung 72 wird radial innen von einem inneren Wandabschnitt 74 des ersten Rohrkörpers 60' begrenzt. Dieser Wandabschnitt 74 bildet ein Führungsmittel 76 für den zweiten Rohrkörper 64 und vor allem für den Zwischenabschnitt 46, 48, welcher mittels des zweiten rohrförmigen Körpers 64 in die zylindrische Ausnehmung 72 eingestülpt wurde. Der Wandabschnitt 74 erstreckt sich wie aus Figur 8 ersichtlich vorteilhafterweise in Längsrichtung 16 über das Ende 62 der radial äußeren Wandung des ersten Rohrkörpers 60' hinaus, so dass dort in vorteilhafter Weise ein Bereich 78 des schlauchförmigen Bahnabschnitts 36 geführt wird und wohldefiniert angeordnet ist.

Nachdem alle Rohrkörper 60, 64 entfernt worden sind, kann die in Figur 9 dargestellte gefaltete Konfiguration in einer Richtung 80 quer zur Längsrichtung 16 in im Wesentlichen ebene Konfiguration, so wie in den Figuren 3 und 4 im Schnitt und in Figur 10 in perspektivischer Ansicht dargestellt, gebracht werden. Diese eben gefaltete Konfiguration könnte beispielsweise um in Figur 10 angedeutete Längs- oder Querachsen 82, 84 weiter gefaltet oder zu Transport- oder Lagerzwecken aufgerollt werden, wenn sich dies als wünschenswert erweist.

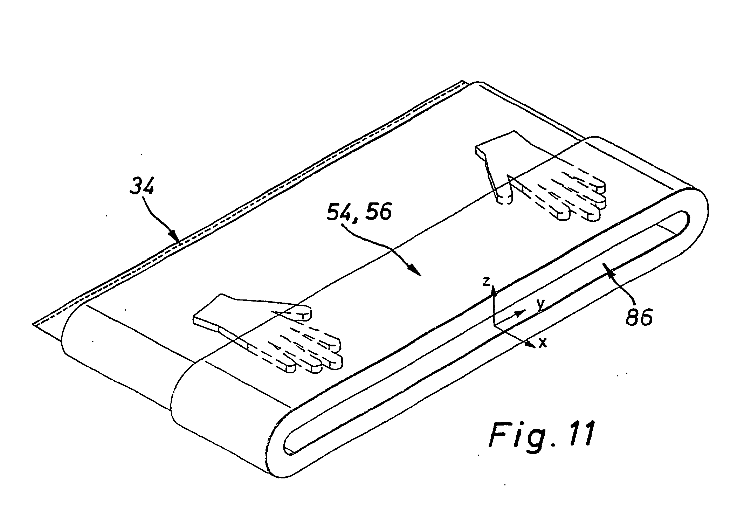

Schließlich zeigt Figur 11 eine schematische perspektivische Ansicht des in ebene Konfiguration gefalteten Bezugs. Man erkennt das von einer flüssigkeitsdichten Bodennaht gebildete geschlossene zweite Ende 34 und den umgeschlagenen Randabschnitt 54, der eine Anfasshilfe 56 bildet, so wie dies durch die abgebildeten Hände dargestellt ist, welche einen bestimmungsgemäßen Eingriff mit der flachen Hand unter den umgeschlagenen Randabschnitt 54 suggerieren. Ferner erkennt man die erfindungsgemäß einzige Öffnung 86 zum Überstülpen über einen Tisch oder eine Extremität eines Patienten.

## Patentansprüche

1. Gefalteter medizinischer Instrumententischbezug (30) zur Verwendung im Operationssaal, mit einem schlauchförmigen Abschnitt (36) mit wenigstens einem ersten offenen Ende (32) zum Aufziehen auf den Instrumententisch (2) und mit einem zweiten geschlossenen Ende (34), wobei der schlauchförmige Abschnitt (36) wenigstens einmal quer zu einer Längsrichtung (16) gefaltet ist, und wobei das zweite Ende (34) einen Anschlag (53) beim Aufziehen des Bezugs auf den Instrumententisch bildet, und mit einer Handhabungshilfe (56) im Bereich des ersten offenen Endes (32) zum Ergreifen des Bezugs (30) mit der Hand eines Benutzers beim Aufziehen auf den Instrumententisch (2), **dadurch gekennzeichnet, dass** ein Zwischenabschnitt (46, 48), der in Längsrichtung (16) zwischen einem das zweite Ende (34) bildenden Endabschnitt (38, 40) und dem ersten offenen Ende (32) angeordnet ist, nach innen in die schlauchförmige Struktur (36) hineingestülpt ist, so dass der Tisch (2) in die von dem hineingestülpten Zwischenabschnitt (46, 48) gebildete Öffnung (49, 86) einführbar ist und der hineingestülpte Zwischenabschnitt (46, 48) beim Aufziehen des Bezugs (30) auf den Tisch (2)entfaltbar ist, wenn der Tisch (2) beim Aufziehen gegen das zweite Ende (34) anliegt und dass die Handhabungshilfe von einem Randabschnitt (54) im Bereich des offenen Endes (32) des Bezugs gebildet ist, der nach außen in Richtung des geschlossenen Endes (34) umgefaltet ist und so eine Falte zum Eingreifen mit der Hand eines Benutzers bildet.

2. Bezug nach Anspruch 1, **dadurch gekennzeichnet, dass** der Randabschnitt (54) umlaufend umgefaltet ist und den gefalteten Bezug (30) **dadurch** in eben gefalteter Konfiguration hält.

3. Bezug nach Anspruch 1 oder 2, **dadurch gekennzeichnet, dass** der Randabschnitt (54) in Längsrichtung etwa 0,2 bis 0,5 der Länge der gefalteten Konfiguration erstreckt ist.

4. Bezug nach einem der vorstehenden Ansprüche, **dadurch gekennzeichnet, dass** mehrere Zwischenabschnitte (46, 48) zwischen dem das zweite Ende (34) bildenden Endabschnitt (38, 40) und dem ersten offenen Ende (32) angeordnet und nach innen in die schlauchförmige Struktur hineingestülpt sind.

5. Bezug nach einem der vorstehenden Ansprüche, **dadurch gekennzeichnet, dass** der eingestülpte Zwischenabschnitt (46, 48) mit seinem durch die Einstülpung gebildeten entfaltbaren Ende (50, 52) innerhalb der schlauchförmigen Struktur (36) bis wenigstens nahezu an den Anschlag (53) am zweiten Ende (34) des Bezugs erstreckt ist.

6. Bezug nach einem der vorstehenden Ansprüche, **dadurch gekennzeichnet, dass** auf wenigstens einer Seite eine absorbierende Oberfläche, insbesondere auf Vliesstoffbasis, vorgesehen ist.

7. Verfahren zum Falten eines medizinischen Instrumententischbezugs zur Verwendung im Operationssaal nach einem oder mehreren der vorstehenden Ansprüche, **gekennzeichnet durch** folgende Verfahrensschritte:
- Herstellen eines schlauchförmigen Bahnabschnitts (36),
- Einstülpen eines Zwischenabschnitts (46, 48), der in Längsrichtung (16) zwischen einem das zweite, geschlossene Ende (34) bildenden Endabschnitt (38, 40) und dem ersten offenen Ende (32) angeordnet ist, nach innen in die schlauchförmige Struktur hinein, so dass der Zwischenabschnitt (46, 48) eine Öffnung (49, 86) zum Einführen des Instrumententischs bildet,
- Umfalten des Randabschnitts (54) im Bereich des ersten offenen Endes (32) nach außen in Richtung des geschlossenen Endes (34).

8. Verfahren nach Anspruch 7, **dadurch gekennzeichnet, dass** zum Herstellen des schlauchförmigen Bahnabschnitts (36) Längsabschnitte von einer in Längsrichtung (16) geförderten endlosen schlauchförmigen Bahn abgetrennt werden.

9. Verfahren nach Anspruch 7 oder 8 **dadurch gekennzeichnet, dass** ausgehend von einem schlauchförmigen Bahnabschnitt (36) dieser Bahnabschnitt (36) auf einen ersten Rohrkörper (60) aufgezogen wird, so dass er über den Rohrkörper (60) vorsteht, und dass mittels eines zweiten Rohrkörpers (64) geringfügig geringeren Durchmessers als der erste ausgehend von dem überstehenden Teil des schlauchförmigen Bahnabschnitts (36) der Zwischenabschnitt (46, 48) in die schlauchförmige Struktur hineingestülpt wird, indem der zweite Rohrkörper (64) unter Mitnahme des Zwischenabschnitts (46, 48) in den ersten hineingeschoben wird.

10. Verfahren nach Anspruch 9, **dadurch gekennzeichnet, dass** der schlauchförmige Bahnabschnitt (36) über den ersten Rohrkörper (60) vorsteht, und dass der überstehende Teil den Zwischenabschnitt (46, 48) bildet.

11. Verfahren nach Anspruch 9 oder 10, **dadurch gekennzeichnet, dass** der zweite Rohrkörper (64) beim Hineinschieben in den ersten Rohrkörper (60) radial innen geführt wird.

12. Verfahren nach Anspruch 11, **dadurch gekennzeichnet, dass** die Führung durch ein radial inneres Führungsmittel (76) gebildet wird, welches Teil des ersten Rohrkörpers (60) ist.

13. Verfahren nach Anspruch 12, **dadurch gekennzeichnet, dass** das innere Führungsmittel (76) von einem inneren Wandabschnitt (74) des ersten Rohrkörpers (60') gebildet ist, der einen zylindrischen Einsteckschlitz (72) für den zweiten Rohrkörper (64) von radial innen begrenzt.

14. Verfahren nach einem der Ansprüche 7 bis 13, **dadurch gekennzeichnet, dass** beim Einschieben des zweiten Rohrkörpers (64) in den ersten Rohrkörper (60) ein Endanschlag (68) zur Begrenzung dieser Bewegung verwendet wird.

15. Verfahren nach einem der Ansprüche 7 bis 14, **dadurch gekennzeichnet, dass** nach dem Hineinstülpen des Zwischenabschnitts (46, 48) der Anschlag (53) für den Tisch gebildet wird.

16. Verfahren nach einem der vorstehenden Ansprüche 7 bis 15, **dadurch gekennzeichnet, dass** der Anschlag (53) durch Schließen des zweiten Endes (34) hergestellt wird.

## Claims

1. Folded medical instrument table cover (30) for use in the operating room, comprising a tubular portion (36) with at least one first open end (32) for pulling onto the instrument table (2) and a second, closed end (34), the tubular portion (36) being folded at least once transversely to a longitudinal direction (16) and the second end (34) forming a stop (53) when pulling the cover onto the instrument table, and comprising a handling aid (56) in the region of the first open end (32) allowing a user manually to grasp the cover (30) when pulling it onto the instrument table (2), **characterised in that** an intermediate portion (46, 48), which is disposed in the longitudinal direction (16) between an end portion (38, 40) forming the second end (34) and the first open end (32), is turned inwardly into the tubular structure (36) such that the table (2) can be inserted into the opening (49, 86) formed by the inwardly-turned intermediate portion (46, 48), and the inwardly-turned intermediate portion (46, 48) can be unfolded when the cover (30) is pulled onto the table (2) when the table (2) abuts the second end (34) while the cover is being applied, and **in that** the handling aid (56) is formed by an edge portion (54) in the region of the open end (32) of the cover, which is outwardly folded in the direction of the closed end (34) to form a fold for engagement by the hand of a user.

2. Cover according to claim 1, **characterised in that** the edge portion (54) is folded peripherally and therefore holds the folded cover (30) in a flatly folded configuration.

3. Cover according to either claim 1 or claim 2, **characterised in that** the edge portion (54) has a longitudinal extent of approximately 0.2 to 0.5 of the length of the folded configuration.

4. Cover according to any one of the preceding claims, **characterised in that** a plurality of intermediate portions (46, 48) are disposed between the end portion (38, 40) forming the second end (34) and the first open end (32) and are turned inwardly into the tubular structure.

5. Cover according to any one of the preceding claims, **characterised in that** the unfolding end (50, 52), formed by inward turning, of the inwardly-turned intermediate portion (46, 48) extends within the tubular structure (36) at least approximately up to the stop (53) at the second end (34) of the cover.

6. Cover according to any one of the preceding claims, **characterised in that** an absorbing surface, in particular a nonwoven-based surface, is provided on at least one side.

7. Method for folding a medical instrument table cover for use in an operating room according to one or more of the preceding claims, **characterised by** the following method steps:
- producing a tubular web portion (36),
- turning an intermediate portion (46, 48), which is disposed in the longitudinal direction (16) between an end portion (38, 40) forming the second, closed end (34) and the first, open end (32), inwardly into the tubular structure, such that the intermediate portion (46, 48) forms an opening (49, 86) for inserting the instrument table,
- folding the edge portion (54) in the region of the first open end (32) outwardly in the direction of the closed end (34).

8. Method according to claim 7, **characterised in that** longitudinal portions are separated from an endless tubular web conveyed in the longitudinal direction (16) to produce the tubular web portion (36).

9. Method according to either claim 7 or claim 8, **characterised in that**, starting from a tubular web portion (36), this web portion (36) is drawn over a first tubular body (60) so as to protrude beyond the tubular body (60), and **in that** the intermediate portion (46, 48) is turned into the tubular structure, starting from the protruding part of the tubular web portion (36) using a second tubular body (64) having a slightly smaller diameter than the first, **in that** the second tubular body (64) is inserted into the first, thus entraining the intermediate portion (46, 48).

10. Method according to claim 9, **characterised in that** the tubular web portion (36) protrudes beyond the first tubular body (60) and **in that** the protruding part forms the intermediate portion (46, 48).

11. Method according to either claim 9 or claim 10, **characterised in that** the second tubular body (64) is guided radially inwardly when it is inserted into the first tubular body (60).

12. Method according to claim 11, **characterised in that** the guide is formed by a radially inner guiding means (76), which is part of the first tubular body (60).

13. Method according to claim 12, **characterised in that** the inner guiding means (76) is formed by an inner wall portion (74) of the first tubular body (60'), which radially inwardly delimits a cylindrical insertion slot (72) for the second tubular body (64).

14. Method according to any one of claims 7 to 13, **characterised in that** an end stop (68) is used for delimiting the insertion of the second tubular body (64) into the first tubular body (60).

15. Method according to any one of claims 7 to 14, **characterised in that** the stop (53) for the table is formed after the intermediate portion (46, 48) has been turned inward.

16. Method according to any one of claims 7 to 15, **characterised in that** the stop (53) is produced by closing the second end (34).

## Revendications

1. Garniture médicale pliée pour table à instruments (30) utilisée en salle d'opération, comportant une partie en forme de tuyau souple (36) ayant au moins une première extrémité ouverte (32) pour enfiler sur la table à instruments (2) et une seconde extrémité fermée (34), la partie en forme de tuyau souple (36) étant pliée au moins une fois transversalement à une direction longitudinale (16) et la seconde extrémité (34) formant une butée (53) lors de l'enfilage de la garniture sur la table à instruments, et ayant un dispositif d'aide au maniement (56) dans la zone de la première extrémité ouverte (32) afin de saisir la garniture (30) avec la main d'un utilisateur lors de l'enfilage sur la table à instruments (2),
**caractérisée en ce qu'**une partie intermédiaire (46, 48), qui est agencée dans la direction longitudinale (16) entre une partie d'extrémité (38, 40) formant la seconde extrémité (34) et la première extrémité ouverte (32), est retournée vers l'intérieur dans la structure en forme de tuyau souple (36), de sorte que la table (2) peut être introduite dans l'ouverture (49, 86) formée par la partie intermédiaire retournée vers l'intérieur (46, 48) et que la partie intermédiaire retournée vers l'intérieur (46, 48) peut être dépliée lors de l'enfilage de la garniture (30) sur la table (2), lorsque la table (2) repose contre la seconde extrémité (34) lors de l'enfilage, et **en ce que** le dispositif d'aide au maniement est formé par une partie de bord (54) dans la zone de l'extrémité ouverte (32) de la garniture, laquelle partie de bord est repliée vers l'extérieur dans la direction de l'extrémité fermée (34) et forme ainsi un pli pour saisir avec la main d'un utilisateur.

2. Garniture selon la revendication 1, **caractérisée en ce que** la partie de bord (54) est repliée tout autour et maintient ainsi la garniture pliée (30) dans une configuration de pliage plat.

3. Garniture selon la revendication 1 ou 2, **caractérisée en ce que** la partie de bord (54) s'étend dans la direction longitudinale sur environ 0,2 à 0,5 de la longueur de la configuration pliée.

4. Garniture selon l'une des revendications précédentes, **caractérisée en ce que** plusieurs parties intermédiaires (46, 48) sont agencées entre la partie d'extrémité (38, 40) formant la seconde extrémité (34) et la première extrémité ouverte (32) et sont retournées vers l'intérieur dans la structure en forme de tuyau souple.

5. Garniture selon l'une des revendications précédentes, **caractérisée en ce que** la partie intermédiaire (46, 48) retournée vers l'intérieur s'étend avec son extrémité (50, 52) dépliable formée par le retournement intérieur à l'intérieur de la structure en forme de tuyau souple (36) au moins jusqu'à proximité de la butée (53) située au niveau de la seconde extrémité (34) de la garniture.

6. Garniture selon l'une des revendications précédentes, **caractérisée en ce qu'**il est prévu une surface absorbante, notamment à base de non-tissé, sur au moins une face.

7. Procédé de pliage d'une garniture médicale pour table à instruments utilisée en salle d'opération selon l'une ou plusieurs des revendications précédentes, **caractérisé par** les étapes suivantes :
- fabrication d'une partie de bande en forme de tuyau souple (36),
- retournement d'une partie intermédiaire (46, 48), laquelle est agencée dans la direction longitudinale (16) entre une partie d'extrémité (38, 40) formant la seconde extrémité fermée (34) et la première extrémité ouverte (32), vers l'intérieur dans la structure en forme de tuyau souple, de telle façon que la partie intermédiaire (46, 48) forme une ouverture (49, 86) pour introduire la table à instruments,
- repli de la partie de bord (54) dans la zone de la première extrémité ouverte (32) vers l'extérieur dans la direction de l'extrémité fermée (34).

8. Procédé selon la revendication 7,
**caractérisé en ce que**, pour fabriquer la partie de bande (36) en forme de tuyau souple, on coupe des parties longitudinales d'une bande sans fin en forme de tuyau souple transportée dans la direction longitudinale (16).

9. Procédé selon la revendication 7 ou 8,
**caractérisé en ce que**, à partir d'une partie de bande en forme de tuyau souple (36), cette partie de bande (36) est enfilée sur un premier corps tubulaire (60) de manière à dépasser au-dessus du corps tubulaire (60), et **en ce que**, au moyen d'un second corps tubulaire (64) de diamètre légèrement inférieur au premier, à partir de la partie dépassant de la partie de bande en forme de tuyau souple (36), la partie intermédiaire (46, 48) est retournée vers l'intérieur dans la structure en forme de tuyau souple en poussant le second corps tubulaire (64) dans le premier tout en entraînant la partie intermédiaire (46, 48).

10. Procédé selon la revendication 9,
**caractérisé en ce que** la partie de bande en forme de tuyau souple (36) dépasse au-dessus du premier corps tubulaire (60) et **en ce que** la partie qui dépasse forme la partie intermédiaire (46, 48).

11. Procédé selon la revendication 9 ou 10,
**caractérisé en ce que** le second corps tubulaire (64) est guidé radialement à l'intérieur lors de l'insertion dans le premier corps tubulaire (60).

12. Procédé selon la revendication 11,
**caractérisé en ce que** le guidage est formé par un moyen de guidage radial interne (76), lequel fait partie intégrante du premier corps tubulaire (60).

13. Procédé selon la revendication 12,
**caractérisé en ce que** le moyen de guidage interne (76) est formé par une partie de paroi interne (74) du premier corps tubulaire (60'), laquelle délimite radialement à l'intérieur une fente d'insertion cylindrique (72) pour le second corps tubulaire (64).

14. Procédé selon l'une des revendications 7 à 13,
**caractérisé en ce que**, lors de l'insertion du second corps tubulaire (64) dans le premier corps tubulaire (60), on utilise une butée de fin de course (68) afin de limiter ce mouvement.

15. Procédé selon l'une des revendications 7 à 14,
**caractérisé en ce que** la butée (53) pour la table est formée après le retournement intérieur de la partie intermédiaire (46, 48).

16. Procédé selon l'une des revendications précédentes 7 à 15,
**caractérisé en ce que** la butée (53) est fabriquée par fermeture de la seconde extrémité (34).
